# EUROPEAN PATENT SPECIFICATION

(11) **EP 4 408 290 B1**
(45) Date of publication and mention of the grant of the patent: **24.09.2025**
(21) Application number: 22789605.7
(22) Date of filing: 29.09.2022
(51) Int. Cl.: A61B 6/00, G01R 33/54, G01R 33/56, G06T 7/00, G06T 7/70, A61B 6/46

(54) **MEDICAL IMAGING AND ANALYSIS METHOD**
MEDIZINISCHES ABBILDUNGS- UND ANALYSEVERFAHREN
PROCÉDÉ D'IMAGERIE ET D'ANALYSE MÉDICALE

(30) Priority: 01.10.2021 EP 21200513
(43) Date of publication of application: 07.08.2024
(73) Proprietor: Koninklijke Philips N.V., 5656 AG Eindhoven (NL)
(72) Inventor: DESHPANDE, Hrishikesh Narayanrao, 5656 AG Eindhoven (NL); BUELOW, Thomas, 5656 AG Eindhoven (NL); SAALBACH, Axel, 5656 AG Eindhoven (NL); HARDER, Tim Philipp, 5656 AG Eindhoven (NL); GOTMAN, Shlomo, 5656AG Eindhoven (NL); COETSER, Edna, 5656AG Eindhoven (NL)
(74) Representative: Philips Intellectual Property & Standards
(86) International application number: PCT/EP2022/077123
(87) International publication number: WO 2023/052509

(56) References cited:
- ZHANG LE ET AL: "Semi-supervised Assessment of Incomplete LV Coverage in Cardiac MRI Using Generative Adversarial Nets", 26 September 2017, ADVANCES IN BIOMETRICS : INTERNATIONAL CONFERENCE, ICB 2007, SEOUL, KOREA, AUGUST 27 - 29, 2007 ; PROCEEDINGS; [LECTURE NOTES IN COMPUTER SCIENCE; LECT.NOTES COMPUTER], SPRINGER, BERLIN, HEIDELBERG, PAGE(S) 61 - 68, ISBN: 978-3-540-74549-5, XP047449037
- SAALBACH AXEL ET AL: "Automated anatomy detection in CT localizer images", 2015 IEEE 12TH INTERNATIONAL SYMPOSIUM ON BIOMEDICAL IMAGING (ISBI), IEEE, 16 April 2015 (2015-04-16), pages 1151 - 1154, XP033179656, DOI: 10.1109/ISBI.2015.7164076
- DESHPANDE HRISHIKESH ET AL: "Deep Learning Based Segmentation of Body Parts in CT Localizers and Application to Scan Planning", 2020 IEEE 17TH INTERNATIONAL SYMPOSIUM ON BIOMEDICAL IMAGING (ISBI), IEEE, 3 April 2020 (2020-04-03), pages 1130 - 1133, XP033774163, DOI: 10.1109/ISBI45749.2020.9098429

## Description

### FIELD OF THE INVENTION

This invention relates to a method for analyzing survey image data within a medical imaging procedure.

### BACKGROUND OF THE INVENTION

Within medical imaging, planning of clinical scans is routinely performed based in part on an initial Surview ("survey view") scan. The survey scan is typically lower resolution than the full scan, and thus can be captured relatively quickly, and (in the case of irradiating modalities), applies a lower radiative dose on the patient. The survey scan can then be used to set parameters of the scan protocol to be followed for the subsequent diagnostic scan, including for example the boundaries of the scan range to be used.

One medical imaging modality in which survey scans are routinely used is computed tomography (CT) imaging (e.g. x-ray CT imaging).

Within CT imaging, in addition to dual 2D survey imaging (frontal and lateral), in the state of the art it is also possible to acquire 3D survey images at considerably lower dose than a typical full 3D scan. Depending upon the anatomy under consideration, a scan has to be planned with respect to specific anatomical landmarks in the survey images in order to ensure that the target anatomy is fully covered in the acquired field-of-view (FOV).

Patient positioning and FOV configuration have a direct impact on the image quality, and hence on the diagnostic value of the images acquired. Accurately planning the scanning parameters to correctly image a target anatomy remains a challenging and time consuming task and requires a qualified technologist.

If the 2D or 3D survey image does not entirely cover the target anatomy in the given FOV, the start and end position of the scan must be adjusted manually by the technologist, based on a mental estimate, to seek to capture the full anatomy in the subsequent diagnostic scan. This is inherently error prone. Errors in the estimation, or deviations from acquisition guidelines, could lead to significant quality deficiencies, such as incomplete images of the target anatomy, in which a portion of the anatomy is missed. If the target anatomy is not entirely covered in the imaged FOV, the diagnostic image scan could cause erroneous diagnosis, e.g. if the missed areas of the anatomy contain pathologies. Furthermore, re-examination becomes necessary, which is inefficient and increases patient dose.

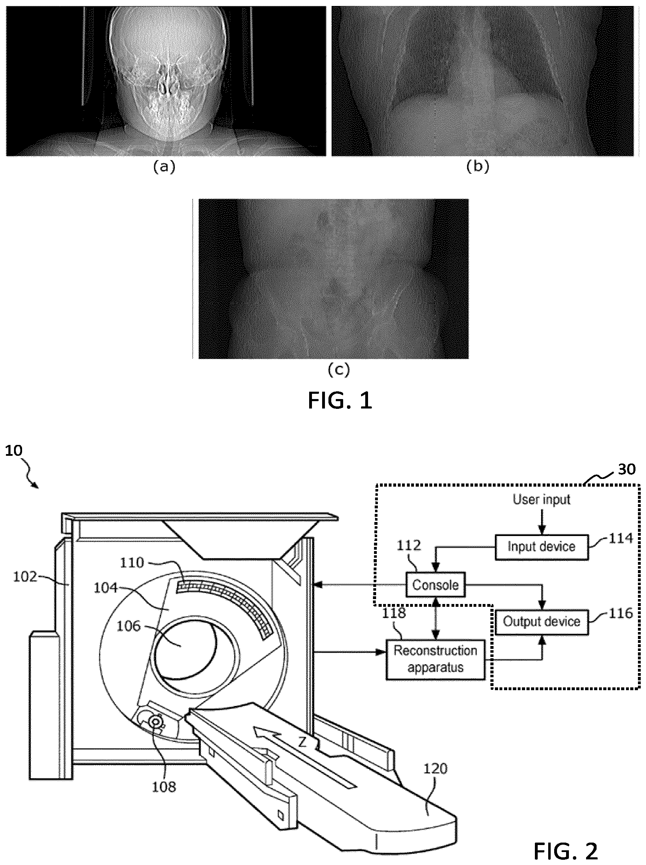

For illustration, Fig. 1 shows some examples of CT images in which a portion of the target anatomy lay outside of the imaged field of view. Fig. 1(a) shows a partially imaged view of the head; Fig. 1(b) shows an image of the chest in which an upper region of the lungs has been excluded from the FOV; Fig. 1(c) shows an image of the pelvis with a lower pelvic area outside of the image FOV.

Zhang Le at al: "Semi-supervised Assessment of Incomplete LV Coverage in Cardiac MRI Using Generative Adversarial Nets" [26 September 2017, Advances in Biometrcis: International Conference, ICB 2007, Seoul, Korea, August 27-29, 2007; Proceedings; Lecture Notes in Computer Science; Springer, Berlin, Heidelberg, page(s) 61-68, XP047449037, ISBN: 978-3-540-74549-5] describes a semi-supervised method to check the coverage of left ventricle from cardiac magnetic resonance images by using generative adversarial networks.

A technical solution capable of addressing one or more of the above-identified problems would be of value.

### SUMMARY OF THE INVENTION

The invention is defined by the claims.

According to examples in accordance with an aspect of the invention, there is provided a computer implemented method comprising:
receiving first image data of a patient anatomy from a medical imaging apparatus, wherein the first image data is 2D or 3D image data, and wherein the first image data is data acquired in accordance with a first scan protocol comprising a first set of scan parameters for the imaging apparatus, wherein the first scan protocol is for acquisition of image data which covers a first FOV;
applying anatomical image analysis to the first image data to detect at least a portion of a defined target anatomy in the image data;
performing a coverage check comprising determining from the image analysis whether the defined target anatomy is fully contained within the first image data;
generating a data representation of the result of the coverage check;
determining a second scan protocol defining a second set of scan parameters for the imaging apparatus, and wherein the second scan protocol is for acquiring image data which covers a second FOV, wherein the second FOV is the same as or different to the first FOV;
controlling a user interface to display an indication of the result of the coverage check, and wherein the method further comprises receiving a user input from the user interface following display of the coverage check, and wherein the second FOV of the second scan protocol is determined in dependence upon the user input; and
acquiring second image data of the patient anatomy in accordance with the second scan protocol, the second image data covering the second FOV, wherein the second image data is 2D or 3D image data.

Thus, embodiments of the invention are based on acquiring first imaging data, which may be survey scan data, and processing the data with an anatomical analysis procedure to identify the target anatomy. From this, a check can be performed as to whether a pre-defined target anatomy is fully covered within the image data (i.e. if the first FOV fully covers the whole of the target anatomy, or if a portion may be missing). A result of the coverage check can then be used in different ways, as will be outlined further below. Second image data is then subsequently applied, which has a second scan protocol, which may or may not be adjusted in dependence upon the result of the coverage check and provided user input. The user input could for instance be an instruction to continue with the second scan protocol identical to the first, or it could for instance be an instruction to adjust the scan protocol.

The method is applicable both for 2D image data and 3D image data. In some examples, the first and second image data could both be 3D (i.e. volumetric) image data, e.g. tomographic image data, e.g. CT or MRI image data. In some examples, the first and second image data could both be 2D image data, e.g. X-ray or ultrasound image data. In some examples, the first image data could be 2D image data and the second image data 3D image data, for example where the first image data is a 2D slice acquired using a 3D or volumetric imaging apparatus.

The target anatomy could be a single anatomical object (such as an organ), a portion of an anatomical object, or could be an anatomical region which encompasses a plurality of anatomical objects.

The first image data is for example image data from a survey scan. The second image data is data from a clinical/diagnostic scan. The first image data may have lower spatial resolution than the second image data. The first image data may contain a smaller amount of data than the second image data.

There are at least three main ways in which the method may proceed following execution of the coverage check and these will be briefly outlined now.

According to the second way, the data representation of the result of the coverage check may be communicated to a datastore for storage of the result of the coverage check; and wherein the second image data is stored in the same or a different datastore, associated with the result of the coverage check.

According to the third way, the method may further comprise: responsive to a negative result of the coverage check, determining a proposed adjustment to the first scan protocol so as to acquire an extended FOV, wherein the proposed adjustment is based on the anatomical image analysis, and then either
setting the second scan protocol in accordance with the proposed adjusted first scan protocol, such that the second FOV is set as the extended FOV; or
communicating the proposed adjustment to the first scan protocol and/or the proposed extended FOV to the user interface.

It is noted that these different options for the method flow following the coverage check are not necessarily mutually exclusive, and features of more than one could be combined. For example, the results of the coverage check can be stored in addition to outputting the results to a user interface and/or determining an adjusted scan protocol.

In accordance with at least one set of embodiments, the method may comprise applying anatomical image analysis to identify a spatial extension of, or a boundary of, at least a portion of the anatomy.

Spatial extension may mean dimensions of a volume of the target anatomy or of a cross-section plane of the anatomy which is captured in the image first image data.

The anatomical image analysis may comprise image segmentation, meaning an operation which identifies boundaries of pre-defined anatomical objects or features, or identifies regions of the image data (2D and/or 3D regions) which are occupied by at least a portion of the anatomical object or feature.

In some embodiments, the method may further comprise applying the anatomical image analysis to estimate a spatial extension of the target anatomy beyond at least one boundary of the FOV. In some examples, the method may further comprise controlling a user interface to display a visual representation of said spatial extension. For example the method may comprise estimating the spatial extension, e.g. a length, in one or more directions or dimensions relative to the FOV or the target anatomy, e.g. superior and/or inferior directions relative to the imaged anatomy.

In some embodiments, the coverage check may comprise determining, based on the spatial extension of the target anatomy beyond the FOV, a proposed adjustment to the first scan protocol for acquiring an extended FOV which fully covers the target anatomy.

In some embodiments, the second scan protocol may be determined such that the second FOV is set as the extended FOV.

In some embodiments, the method may comprise: controlling a user interface to display a representation of the proposed adjustment to the first scan protocol and/or proposed extended FOV; generating a prompt on the user interface requesting user approval; receiving a user input from the user interface indicative of approval or non-approval, and acquiring second image data in accordance with the proposed adjusted scan protocol, spanning the extended FOV, only responsive to receipt of a user input indicative of approval.

In other words, the proposed adjustment is sent to the user interface for conformation by the user, and is subsequently utilized for the second scan only if the user indicates approval.

In some embodiments, the method may comprise controlling a user interface to display a visual representation of the proposed extended FOV relative to a rendered view of the first image data (e.g. an outline of a boundary of the extended FOV superposed on top of the first image data); and controlling the user interface to generate a prompt for a user input indicative of approval of the proposed extended FOV or indicative of an amendment to the proposed extended FOV, via operation of a user control. The method may then further comprise either: determining the second scan protocol such that the second FOV is set as the extended FOV, responsive to receipt of user approval from the user-interface; or determining the second scan protocol such that the second FOV is set as a user-amended FOV, wherein the user-amended FOV is defined based on a received user input indicative of an amendment to the extended FOV.

The scan parameters may include boundaries of a scan range along at least one scan axis of the medical imaging apparatus, and wherein the FOV is defined at least in part by the boundaries of said scan range. In other words, adjustment of the FOV can be achieved by adjusting the scan range. The proposed adjustment to the first scan protocol which is generated by the method in accordance with certain embodiments, may include a proposed adjustment to the boundaries of the scan range long one or more scan axes.

The aforementioned step of estimating the spatial extension of the target anatomy outside of the FOV may comprise estimating an outline of a boundary of at least the portion of the target anatomy which lies outside of the FOV. The method may further comprise generating a visual depiction of said outline relative to the first image data on a display of a user interface.

In accordance with any embodiments of the invention, the scan parameters of the first and second scan protocols may include boundaries of a scan range along at least one scan axis of the medical imaging apparatus, and wherein the first FOV and second FOV are each defined at least in part by the boundaries of said scan range.

The scan parameters of the first and second scan protocols may further include a physical placement of the subject/patient anatomy relative to the medical imaging apparatus. For example, this may comprise a physical positioning of a subject support, table or couch relative to the imaging apparatus (e.g. in the case of MRI or CT imaging). In other cases it could comprise a physical placement of an imaging aperture or probe relative to a patient, e.g. in the case of X-ray or ultrasound imaging.

The proposed adjustment to the first scan protocol may comprise a proposed adjustment to said boundaries of the scan range along the at least one scan axis. In other words, it may comprise an adjustment to a start and/or end of scan range. The scan range may include an angular range about a rotation axis of the medical imaging apparatus. The scan range may include an axial range along an axial direction of the medical imaging apparatus.

In some embodiments, the data representation may be communicated to a datastore for storage of the result of the coverage check; and wherein the method further comprises performing a quality assessment comprising deriving a quality indicator for the acquired second image data based on the result of the coverage check associated with the image data.

There are different options for the image analysis operation. In some embodiments, the image analysis operation applies anatomical image segmentation.

In some examples, the performing the image analysis operation and/or the coverage check comprises application of a machine learning model.

In some embodiments, the machine learning model is configured to: receive, as an input, image data representing only a portion of an anatomical object, and
generate, as an output, an estimate of at least a dimensional extent of a remainder of the anatomical object outside a field of the image data; and/or
generate, as an output, an estimation of a boundary of the remainder of the anatomical object outside a field of the image data.

The model may be trained with training data comprising cropped images of the anatomical object. For example the machine learning model may be trained using training data entries which each comprise
a first data input for the model comprising a first, cropped image of the anatomy in which a section of the image has been removed so that the image depicts only a portion of the anatomical object, and
a ground truth for the model comprising at least voxel-wise anatomical labelling for voxels of a second, non-cropped version of the image, which depicts the whole of the anatomical object.

In other words, the ground truth for the model comprises labelled anatomical voxels even outside the simulated/cropped field-of-view of the first image.

In other words, the training data entries each comprise a version of the image which covers the whole of the anatomy of interest, as well as version of the same image which covers only a portion of the anatomy of interest and voxel-wise annotations of the image with labelled anatomical voxels outside the field-of-view.

The ground-truth will still contain the voxel-wise annotation for the entire anatomy, even though a portion of the target anatomy is simulated to be outside the FOV.

Although voxels are referred to above, the images may be 2D, in which case they comprise pixels instead of voxels.

A cropped image means an incomplete or partial image.

Another aspect of the invention provides a processing arrangement which comprises an input/output for connecting in use to a medical imaging apparatus; and one or more processors. The one or more processors are adapted to:
receive, at the input/output, first image data of a patient anatomy from a medical imaging apparatus, wherein the first image data is 2D or 3D image data, and wherein the first image data is data acquired in accordance with a first scan protocol comprising a first set of scan parameters for the imaging apparatus, wherein the first scan protocol is for acquisition of image data which covers a first FOV;
apply anatomical image analysis to the first image data to detect at least a portion of a defined target anatomy in the image data;
perform a coverage check comprising determining from the image analysis whether the defined target anatomy is fully contained within the first image data;
generate a data representation of the result of the coverage check;
determine a second scan protocol defining a second set of scan parameters for the imaging apparatus, and wherein the second scan protocol is for acquiring image data which covers a second FOV, wherein the second FOV is the same as or different to the first FOV;
control a user interface to display an indication of the result of the coverage check;
receive a user input from the user interface following display of the coverage check, and wherein the second FOV of the second scan protocol is determined in dependence upon the user input; and
output at the input/output a control signal for controlling the imaging apparatus to acquire second image data of the patient anatomy in accordance with the second scan protocol, the second image data covering the second FOV, wherein the second image data is 2D or 3D image data.

A further aspect of the invention provides a system, comprising: a medical imaging apparatus; and a processing arrangement in accordance with any example or embodiment outlined above, or in accordance with any claim of this application.

In some embodiments, the medical imaging apparatus is a tomographic imaging apparatus, such as an X-ray CT imaging apparatus or an MRI imaging apparatus.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows examples of medical images acquired with an erroneously places FOV, leading to incomplete imaging of a target anatomy;
Fig. 2 shows an exemplary medical imaging apparatus;
Fig. 3 shows an example system and processing arrangement in accordance with one or more embodiments of the invention;
Fig. 4 shows an example graphical user interface display screen displaying a result of an anatomy coverage check, in the form of a message indicating a negative (non-coverage) result;
Fig. 5 shows an example graphical user interface display screen displaying a proposed adjusted FOV responsive to a negative anatomy coverage check, where the proposed adjusted FOV is in the form of a graphically displayed outline of a boundary of the extended FOV relative to initial image data; Fig. 6 shows the same graphical user interface display screen as Fig. 5, except in addition showing a segmented outlined of a portion of a boundary of a target anatomy which lies outside of the first FOV;
Fig. 7 shows the same graphical user interface display screen as Fig. 5 and 6 with additional indication of different organs present within the FOV listed in a text form;
Fig. 8 illustrates creation of training data for training a machine learning model to perform an anatomy coverage check, the training data comprising clipped version of a ground truth image, the clipped versions clipped to exclude at least a portion of the depicted anatomy from the image field;
Fig. 9 illustrates the result of application of a machine learning algorithm which has been trained to perform the anatomy coverage check; and
Figs. 10-11 illustrate further results of application of a machine learning algorithm which has been trained to perform the anatomy coverage check, where the training was specifically for CT imaging.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for analyzing survey imaging data. The method comprises acquiring first image data with a first imaging protocol coving a first FOV, processing the data with an anatomical analysis program or routine to detect at least a portion of a target anatomy, and performing a coverage check adapted to determine whether the target anatomy is fully covered within the first FOV. Second image data is subsequently acquired in accordance with a second image protocol defining a second FOV. The second imaging protocol may be: the same as the first, but wherein the results of the coverage check are stored and linked with the second image data for later use; different to the first and wherein the results of the coverage check are output to a user interface and a user input responsive thereto is used to determine the second scan protocol; or different to the first, but wherein an adjusted second scan protocol is automatically determined.

Various embodiments of the present invention, to be described in detail further below, may, but not necessarily, comprise one or more of the following features.

In some embodiments, the provided system or method aims to alert a user in cases where a target anatomy (such as a target organ) is not entirely within a captured or planned FOV. This could be achieved by employing an image classification approach, for example using a machine learning algorithm such as a deep learning artificial neural network. The analysis may be applied to 2D or 3D survey view images.

In some embodiments, the provided system or method may aim to determine an extent of the partially missing anatomy outside of the FOV captured in the survey image, even if this is beyond the boundary of the image data acquired. The system or method may generate a recommended patient positioning relative to the imaging apparatus and/or recommended image acquisition parameters (such as start and end positions of the scan range in one or more scanning dimensions) so as to improve the anatomy coverage before acquisition of the diagnostic image data set.

In some embodiments, image segmentation or object-detection based deep learning methods may be used for the estimation of the organ-extent, based on input survey image data.

The embodiments of the invention may be applied to multiple different imaging modalities and also to additional applications such as retrospective image quality assessment.

Embodiments of the method of the invention involve receiving image data from a medical imaging apparatus, and may comprise determining scan protocols for acquiring image data covering an adjustable FOV.

The principles of the inventive concept are not limited to use with any particular type of medical imaging apparatus. However, they are most advantageously applicable for tomographic imaging such as x-ray Computed Tomography (CT) scanning and MRI scanning. Embodiments of the invention can be applied for 2D image data and/or 3D image data.

To assist in illustrating the principles of the invention, an exemplary medical imaging apparatus is shown in Fig. 2. The imaging apparatus 10 in this illustration is an x-ray computed tomography (CT) scanner.

The imaging apparatus 10 includes a generally stationary gantry 102 and a rotating gantry 104. The rotating gantry 104 is rotatably supported by the stationary gantry 102 and rotates around an examination region about a longitudinal, axial, or z-axis.

A patient support 120, such as a couch, supports an object or subject such as a human patient in the examination region. The support 120 is configured to move the object or subject for loading, scanning, and/or unloading the object or subject. The support 120 is movable along an axial direction, that is, it is moveable along the direction of the z-axis or the longitudinal axis. Moving the support changes the axial position of the rotating gantry relative to the support (and thus relative to the subject who is supported by it).

A radiation source 108, such as an x-ray tube, is rotatably supported by the rotating gantry 104. The radiation source 108 rotates with the rotating gantry 104 and emits radiation that traverses the examination region 106.

A radiation sensitive detector array 110 subtends an angular arc opposite the radiation source 108 across the examination region 106. The detector array 110 includes one or more rows of detectors that extend along the z-axis direction, detects radiation traversing the examination region 106, and generates projection data indicative thereof.

The rotation of the gantry 104 changes an angular or rotational position of the scanner relative to the subject, and movement of the support along the z-axis changes the axial position of the scanner relative to the subject.

A typical scan will be configured in advance with a scan protocol. A scan protocol comprises a plurality of scanning parameters. The scanning parameters define, among other things, a spatial range of the scan relative to the axial and rotation axes of the scanner. For example, the scan parameters may include boundaries (that is, start and end points) of a scan range along one or more of the axes of the imaging apparatus, for example one or both of the rotational and axial axes. The scan range defines the field of view (FOV) over which imaging data is acquired during the scan. The scanning parameters may typically also include a number of other parameters including for example tube current, tube voltage, scan spatial resolution, scan temporal resolution, fan angle. The resolution parameters may be defined by a speed of rotation of the gantry 104 and the speed of axial movement of the support 120 through the gantry.

A general-purpose computing system or computer serves as an operator console 112 and includes an input device(s) 114 such as a mouse, a keyboard, and/or the like and an output device(s) 116 such as a display monitor, a filmer or the like. The console, input device(s) and output device(s) form a user interface 10. The console 112 allows an operator to control operation of the system 100.

A reconstruction apparatus 118 processes the projection data and reconstructs volumetric image data. The data can be displayed through one or more display monitors of the output device(s) 116.

The reconstruction apparatus 118 may employ a filtered-back-projection (FBP) reconstruction, a (image domain and/or projection domain) reduced noise reconstruction algorithm (e.g., an iterative reconstruction) and/or other algorithm. It is to be appreciated that the reconstruction apparatus 118 can be implemented through a microprocessor(s), which executes a computer readable instruction(s) encoded or embed on computer readable storage medium such as physical memory and other non-transitory medium. Additionally or alternatively, the microprocessor(s) can execute a computer readable instruction(s) carried by a carrier wave, a signal and other transitory (or non, non-transitory) medium.

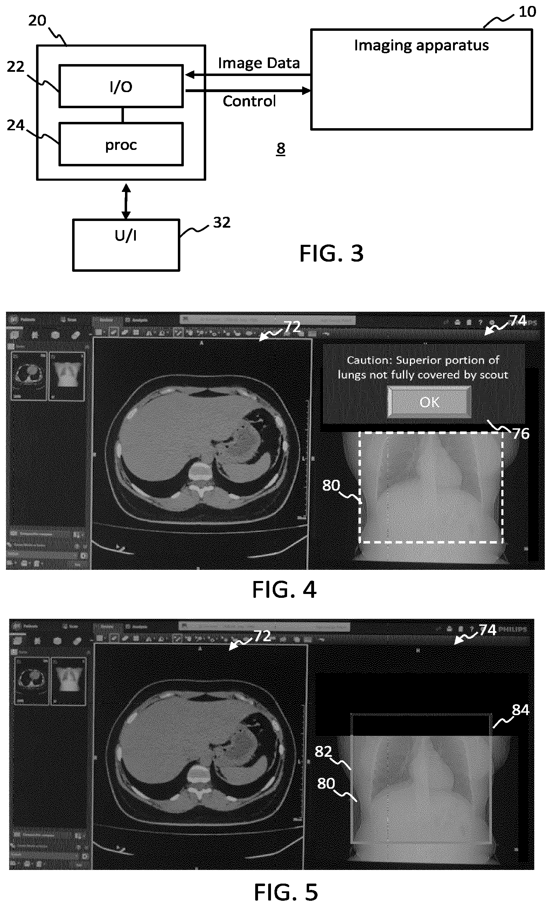

Fig. 3 shows a block diagram of an example system 8 in accordance with one or more embodiments of the present invention. The system comprises a processing arrangement 20 which comprises an input/output (I/O) 22 or communication module for connecting in use to a medical imaging apparatus 10, and further comprises one or more processors ("proc") 24 adapted to carry out steps of a computer implemented method, which will be described below. The system may comprise the medical imaging apparatus, or the medical imaging apparatus may be external to the system and communicatively coupled with it.

The system may further comprise a user interface (UI) 32 communicatively coupled with the processing arrangement 20. The user interface may comprise a display with a screen for displaying a visual output to a user. The user interface may provide a graphical user interface. The user interface may also include means for generating other sensory outputs, such as acoustic output. The user interface may be a computer console comprising a display screen, a user input device, and a processor. The display screen may be touch-screen display, and may thereby integrate the user input device. Alternatively, the user input device may comprise a keyboard and/or pointer device (e.g. mouse pointer).

As mentioned above with reference to Fig. 2, in some cases, the imaging apparatus 10 may comprise its own user interface 30. In some examples, the user interface of the imaging apparatus may be used to perform the role of the user interface 32 of the system. In other examples, the system may comprise its own user interface.

The processing arrangement 20 forms an aspect of the invention by itself. The aforementioned computer-implemented method, which the processing arrangement is configured to implement, also forms another independent aspect of the invention.

An example computer-implemented method in accordance with at least one set of embodiments of the invention will now be outlined in summary, before expounding further on the features and different possible embodiments of the method.

The method comprises receiving first image data of a patient anatomy from a medical imaging apparatus 10, wherein the first image data is 2D or 3D image data. The first image data is data acquired in accordance with a first scan protocol comprising a first set of scan parameters for the imaging apparatus. The first scan protocol is adapted to cause acquisition of image data which covers a first field of view (FOV). In other words, the first set of scan parameters of the first scan protocol cause the imaging apparatus to acquire image data over a first FOV. The FOV in this context means the area over which image data is acquired. Therefore the received first image data consists of image data for the first FOV only in this example. Typically, the scan parameters of the scan protocol include defined boundaries of a scan range along at least one scan axis of the medical imaging apparatus, for example along the axial or longitudinal axis and/or the rotational axis previously mentioned with reference to Fig. 2. The scan range typically defines, at least in part, the FOV over which data is acquired. The FOV in general means a FOV defined relative to the imaging apparatus, e.g. relative to a co-ordinate system of the imaging apparatus.

The method further comprises applying anatomical image analysis to the first image data to detect at least a portion of a defined target anatomy in the image data. For example, anatomical image analysis may comprise anatomical image segmentation to detect boundaries of anatomical objects or regions within the scanned area, and/or detect volumes occupied by anatomical objects or regions in the scanned area.

The method further comprises performing a coverage check comprising determining from the image analysis whether a defined target anatomy is fully contained within the first image data. The target anatomy could be a single anatomical object or feature (such as an organ), a portion of an anatomical object (e.g. a chamber or valve of a heart), or could be an anatomical region which encompasses a plurality of anatomical objects (e.g. a particular upper body region including multiple organs).

The method further comprises generating a data representation of the result of the coverage check. The data representation may be for export from the processing arrangement via the input/output, and/or might be for further use in further processing performed in accordance with the method.

The method further comprises determining a second scan protocol defining a second set of scan parameters for the imaging apparatus, and wherein the second scan protocol is adapted to cause acquisition of image data which covers a second FOV, wherein the second FOV is the same as or different to the first FOV.

The method further comprises acquiring second image data of the patient anatomy in accordance with the second scan protocol, the second image data covering the second FOV, wherein the second image data is 2D or 3D image data.

The first image data may be data acquired in an initial survey scan, which may acquire 2D image data, or may acquire 3D image data. The method is for checking whether the planned scan parameters will result in a FOV which fully captures the target anatomy. The survey scan is used to adjust (if needed) scan parameters before triggering execution of the full diagnostic scan, which typically has higher image resolution than the survey scan, and takes longer to perform. The aforementioned second image data may be data acquired for the full diagnostic scan. The second image data may be 3D (volumetric) image data, or may be 2D image data.

Following acquisition of the first image data, and the execution of the coverage check, there are at least three main ways that the method may progress, which may be summarized as follows.

The first is that the user interface 32 is controlled to display an indication of the result of the coverage check, and wherein the method further comprises receiving a user input from the user interface following display of the coverage check, and where the second FOV of the second scan protocol is determined in dependence upon the user input which is received. The user interface may be controlled to present the user with an option to continue with the first scan protocol with the first FOV for acquiring the subsequent second image data. In this case, the second scan protocol and second FOV is the same as the first. The user interface may also present the user with an option to adjust one or more of the scan parameters of the scan protocol so as to adjust the FOV. In this case, the second scan protocol and second FOV would be different to the first, and adjusted in dependence upon the user's input. In some cases, the scan protocol could be adjusted, so that the second scan protocol is different to the first, but while keeping the second FOV the same (e.g. change radiation dose). As will be outlined in more detail later in this disclosure, the adjustment to the scan protocol for the second image data acquisition may be fully user-defined (e.g. with user-controls provided on the user interface), or may be at least semi-automated (e.g. the method comprises automatically generating one or more proposals for adjusting the scan protocol and the FOV based on the result of the coverage check). In the latter case, the user input might comprise user-indicated acceptance or rejection of the proposed adjustments, or user-indicated alterations to the proposed adjustments.

A second way that the method can progress is that the result of the coverage check is communicated to a datastore for storage of the result of the coverage check, and wherein the second image data is stored in the same or a different datastore, associated with the result of the coverage check. In other words, the coverage check is stored for later reference, and there is generated a data link by which the stored coverage check result can be associated with the corresponding second image data which is acquired after the result is derived. In this case, the second scan protocol and second FOV are the same as the first image protocol and the first FOV, and the coverage check is simply used for subsequent analysis of potential quality of the second image data.

The third way that the method can progress is that the method further comprises, responsive to a negative result of the coverage check (i.e. the target anatomy is not fully covered by the first FOV), determining a proposed adjustment to the first scan protocol, so as to acquire an extended FOV, wherein the proposed adjustment is based on the anatomical image analysis. The proposed adjustment would aim to result in a new FOV which does fully cover the target anatomy. For example, the proposed adjustment could include an adjustment to start and/or ends points of a scan range along one or more scan axes. It could include table motion parameters (such as table speed). The method may then in some cases automatically set the second scan protocol in accordance with the proposed adjusted first scan protocol, such that the second FOV is set as the extended FOV. Alternatively, the method may comprise communicating the proposed adjustment to the first scan protocol and/or the proposed extended FOV to the user interface. The user interface might be controlled such as to permit the user to input a response to the proposed adjustment, e.g. binary acceptance or rejection, or a modification to the proposed adjustment.

With regards to the anatomical image analysis, this may comprise identifying a spatial extension of, or a boundary of, at least a portion of the anatomy. In other words, it may comprise anatomical image segmentation.

If the coverage check has a positive result, then the processing arrangement may simply acquire the second imaging data using the original first scan protocol, optionally after seeking confirmation from the user via the user interface.

To further illustrate the concepts of the invention, the steps of an example implementation will now be outlined in detail, with reference to Figs. 4-7.

As a first step, one or more 2D or 3D survey images are acquired at the start of the examination. These form the previously mentioned first image data. These are generated in accordance with a first scan protocol which results in acquisition of a first imaging FOV relative to the imaging apparatus.

A user interface 32 may be controlled to generate a visual representation of the first image data.

Optionally, in order to improve the accuracy of the prediction, additional information could be acquired for the subsequent processing. This may include relevant acquisition and protocol parameters such as the target anatomy to be imaged, scanning parameters such as the tube voltage and/or current settings, and/or patient demographic information such as age and gender.

The method further comprises applying image analysis (e.g. anatomical image segmentation) to detect the target anatomy in the first image data. A user interface 32 may be controlled to display a visual representation of the anatomy detection. For example, an outline of at least a portion of the detected target anatomy may be shown, or the volume which is detected as being occupied by the target anatomy might be shown highlighted, or a portion of the image FOV which contains the target anatomy might be shown outlined.

Based on processing of the acquired first image data, and optionally also based on one or more of the parameters mentioned above, the method applies a coverage check operation adapted to infer whether the target anatomy is fully covered in the present FOV. As will be explained in more detail below, the coverage check operation may be performed by a trained machining learning model.

A user interface 32 may be controlled to display a visual representation of the result of the coverage check.

In at least some embodiments, if the coverage check returns a negative result (that is, the coverage check determines that the target anatomy is not fully covered by the present FOV), then an alert message may be generated using the user interface 32, for instance a visual and/or auditory alert may be generated.

An example is illustrated in Fig. 4 which shows an example graphical user interface display screen. The screen displays the acquired first image data, in this case first 72 and second 74 survey image views from a CT scanning apparatus. The views may be separately acquired 3D image views, or may be slices extracted from a 3D survey image. The user interface further displays a visual representation of the result of the anatomy detection, e.g. segmentation. In the example of Fig. 5 for example, a region 80 of the image FOV which contains the target anatomy is shown highlighted as a semi-transparent overlay over the relevant portion of the image FOV which contains the target anatomy. For ease of illustration, this is shown highlighted with a white dashed outline, but in practice it may consist for example simply of a color-shaded semi-transparent overlay. In this example, display screen is being controlled to further display a pop-up message 76 indicating a negative (non-coverage) result. In this example for instance, the message alerts the user that a superior portion of the lungs is not covered by the "scout" image (meaning the survey image). The operator may have the option either to ignore the message, or may decide to take action by modifying the planned scan protocol for the diagnostic scan, e.g. to cause adjustment of the FOV.

In some embodiments, the method may further comprise applying the anatomical image analysis to estimate a spatial extension of the anatomical object beyond at least one boundary of the first FOV. This may include estimating a distance outside of the FOV to which the anatomy extends, along with the corresponding position (e.g. superior or inferior), and communicating this to the operator. The operator might be prompted or provided means on the user interface for modifying the scan parameters, for instance to adjust a start and end position of the scan range for the diagnostic scan.

The user interface may be controlled to display a visual representation of the estimated spatial extension of the target anatomy beyond the FOV. An example is illustrated in Fig. 5 which shows the first 72 and second 74 image view from the first (survey) image data, and shows in overlay atop the second image view a visual representation of a detected portion 80 of the imaging FOV which is occupied by the target anatomy, and an outline of a boundary of an estimated spatial extension 84 of the anatomical object beyond at least one boundary of the first FOV. The region 80 might for example be represented by a color-tinted semi-transparent overlay box, optionally with a solid outline. The estimated region 84 occupied by the portion of the target anatomy not covered in the first FOV may be shown as an outline around the estimated region 84, for example in a different color as is used to highlight the region that does contain the detected anatomy.

In some examples, the estimated spatial extension 84 of the target anatomy outside of the FOV may be used to inform a proposed adjusted FOV which would fully capture the target anatomy. In particular, the method may further comprise determining, based on the detected spatial extension 84 of the anatomical object beyond the FOV, a proposed adjustment to the first scan protocol for causing the imaging apparatus to acquire an extended FOV which fully covers the anatomical object.

The adjusted scan protocol may then in some cases be directly implemented, i.e. the second scan protocol is determined such that the second FOV is set as the extended FOV, and the second image acquisition is started.

Alternatively, the operator may be provided an option to accept or reject the proposed adjustment to the field of view for the second scan.

In particular, the method may further comprise: controlling a user interface to display a representation of the proposed adjustment to the first scan protocol and/or proposed extended FOV; generating a prompt on the user interface requesting user approval; receiving a user input from the user interface indicative of approval or non-approval, and acquiring second image data in accordance with the proposed adjusted scan protocol, spanning the extended FOV, only responsive to receipt of a user input indicative of approval.

Optionally, in addition to the option to approve or reject the proposed adjustment, the user might also be provided the option to modify the suggested FOV extension, for instance by changing the dimensions of the proposed adjusted FOV, e.g. as indicated by the highlighted extension region 84 on the user interface.

In other words, the method may further comprise: controlling a user interface to display a visual representation of the proposed extended FOV relative to a rendered view of the first image data; controlling the user interface to generate a prompt for a user input indicative of approval of the proposed extended FOV or amendment to the proposed extended FOV, via operation of a user control; and then either:
- determining the second scan protocol such that the second FOV is set as the extended FOV, responsive to receipt of user approval from the user-interface; or
- determining the second scan protocol such that the second FOV is set as a user-amended FOV, wherein the user-amended FOV is defined based on a received user input indicative of an amendment to the extended FOV.

To amend the proposed adjusted FOV, or the proposed adjusted scan protocol, the user might have the option to drag the outline of a box which highlights on-screen the proposed FOV extension, and/or may have the option to manually configure the scan parameters, such as the start and end positions of the scan range along one or more scan axes.

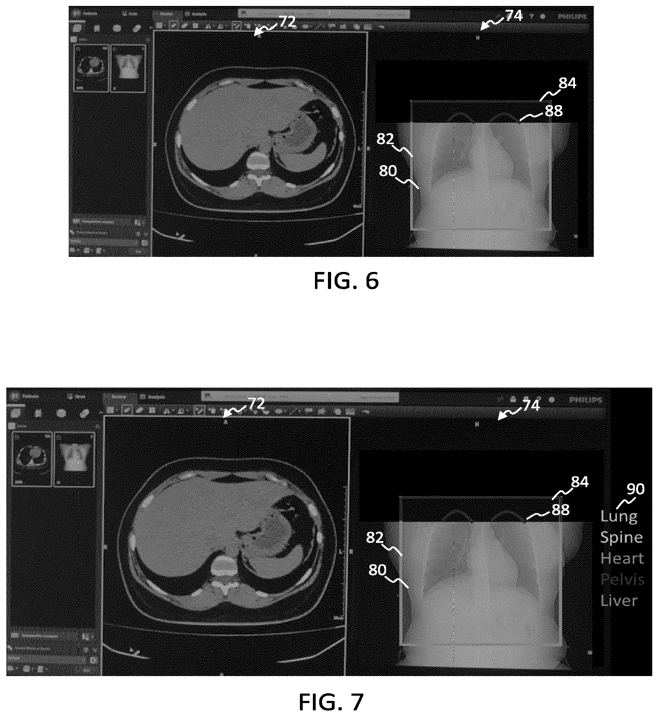

Further to any of the above, in some embodiments, the estimating of the spatial extension of the anatomical object outside of the FOV may comprise estimating an outline of a boundary of at least the portion of the object which lies outside of the FOV, and wherein the method further comprises generating a visual depiction of said outline relative to the first image data on a display of a user interface. An example is illustrated in Fig. 6 where a contour 88 of the estimated continuation of the anatomy outside the FOV is presented on the display as a sketch.

Additionally, in some embodiments, the anatomical image analysis, e.g. anatomical image segmentation, may be used to generate a labelling of different anatomical structures depicted in the first image data FOV. For instance, Fig. 7 shows one example in which the different organs present within the FOV of the first image data is listed in text form. In some examples, a visual indication may further be provided indicative of whether each anatomical structure is fully covered or not fully covered by the first FOV. Advantageously, this could be implemented through means of the formatting of the text, e.g. the text color. For instance, green text would be used to indicate the anatomical structure if completely covered in the FOV, orange to indicate that the anatomical structure is partially outside of the FOV, and red to indicate that the anatomical structure is entirely outside of the FOV.

In a variation on the above-described example, instead of providing the user the option to amend the FOV, in simpler embodiments, the user may simply be presented an option to approve or reject a proposed adjusted FOV.

In a variation on the above-described example, instead of showing the user the proposed adjusted FOV, the processing arrangement may simply automatically acquire the second imaging data using the adjusted FOV without seeking user approval. A user interface is not essential to the invention, any may not be used at all in simpler embodiments.

As mentioned above, instead of, or in addition to, adjusting the scan protocol responsive to the coverage check results (either with user input or without user input), the method may comprise communicating the data representation of the coverage check result to a datastore for storage of the result of the coverage check. This data may subsequently be used for imaging quality analysis. This could be done in some examples retrospectively, at a different time to when the image data is acquired.

In some examples, the method may comprise retrieving the stored second image data; and retrieving the stored result of the coverage check based on a data link which provides a data pointer between storage addresses of the stored second image data and the stored coverage check result for the first image data. The method may further comprise performing a quality assessment comprising deriving a quality indicator for the second image data based on the result of the coverage check associated with the image data. This could be stored for later retrieval. It could be output to a user interface. In either case, the quality indicator is useful in informing a clinician viewing the second image data as to a reliability of the data for performing diagnostic analysis. In cases where quality is low, a clinician may place lower significance on the data, and for example not make major treatment decisions based purely on the image data. If quality is high, the clinician might place greater significance on the image data.

In some embodiments, an analysis operation may be applied to the coverage check results for a plurality of patients, e.g. a whole cohort or population of patients. This can be used to generate statistical information about the quality of imaging, for instance within a group or department. The results might be presented on a graphical user interface display, e.g. in the form of dashboard, to support quality monitoring. In some examples, the quality indicator mentioned above may first be generated for the image data of each of the plurality of patient imaging datasets to be included in the analysis, and then the analysis is performed on the quality indicators for the plurality of datasets.

As discussed above, embodiments are adapted to perform a coverage check comprising determining from the image analysis whether a defined target anatomy is fully contained within the acquired first image data. For this purpose, in accordance with at least one set of embodiments, the method comprises application of a machine learning model, such as a deep learning artificial neural network model. In some examples, the model may be adapted to provide a classification output, e.g. a binary output indicative of whether a target anatomy is covered within the field of view of an input image.

In some embodiments, there may be provided a separate machine learning model for each of a plurality of different possible target anatomies, each specifically trained for that anatomy (e.g. chest, spine, heart, pelvis, abdomen and combined anatomies such as chest-abdomen, abdomen-pelvis and chest-abdomen-pelvis). There could also be different models for different demographic categories, such as different age groups and genders. Thus a bundle of machine learning models may be provided, for each of a set of possible target anatomies, and for a plurality of different demographic classifications. In other examples, there may just be a single machine learning model which has been trained to detect any of a plurality of different target anatomies.

In accordance with at least one set of embodiments, the one or more machine learning models may be configured to: receive, as an input, image data representing only a portion of an anatomy of interest, and generate, as an output, an estimate of at least a dimensional extent of a remainder of the anatomy of interest outside a field of the image data; and/or generate, as an output, an estimation of a boundary of the remainder of the anatomical object outside a field of the image data.

The training data for such a machine learning model could be generated by artificially truncating full image views of certain target anatomies to deliberately exclude portions of the anatomy from the resulting image data. The full, uncropped, image could also be used as the ground-truth for the training. The full version could be segmented in advance of cropping it, and a boundary outline (or annotation mask or mesh) of the target anatomy both within the remaining portion of the cropped image and within the cropped part of the image also stored as Ground Truth.

Thus, in other words, the machine learning model may be trained using training data entries which each comprise: a first data input for the model comprising a cropped image of the anatomy in which a section of the image has been removed so that the image depicts only a portion of the anatomical object, and a ground truth for the model comprising voxel-wise anatomical annotation of the image over a non-cropped image field which includes the whole of the anatomy of interest (i.e. including voxel annotations within the portion of the image which is uncropped and within the portion of the image which is cropped). The annotations for example are obtained from the image segmentation, the output of which may be a voxel-wise indication of whether each voxel is part of an anatomy, and if so which anatomy it is part of.

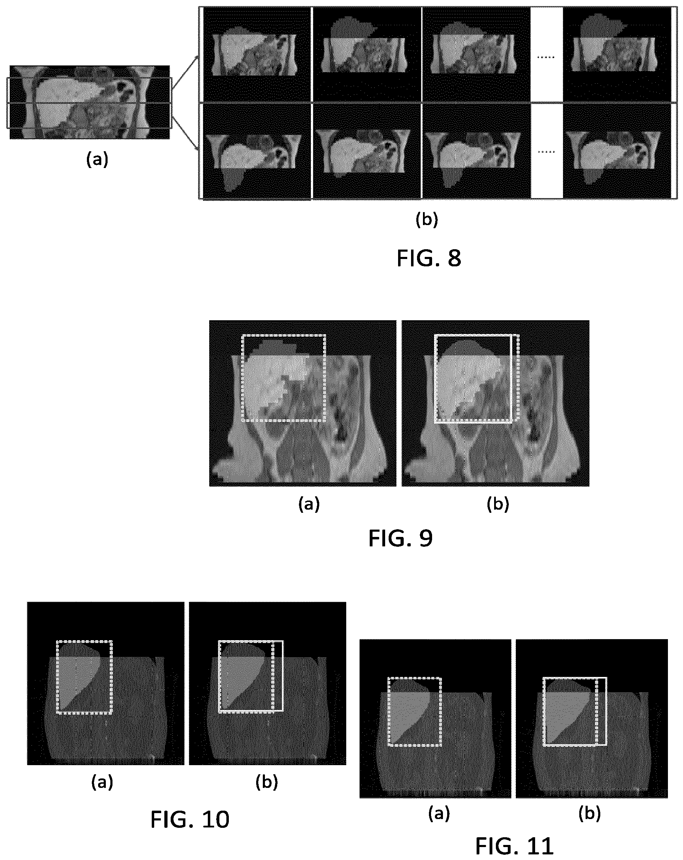

By way of illustration, Fig. 8 illustrates the generation of training data for an example in which the target anatomy is the liver. Fig. 8(a) shows the original full image of the liver, in addition to the segmentation mask for the liver region. Fig. 8(b) illustrates construction of a set of example artificially cropped or clipped images, each cropped to exclude a portion of the liver from the resulting image field. The brighter region in each image is the uncropped part. For the liver, the image could for instance be clipped at the superior or inferior image positions to generate several images with different clipping positions from a single case. While each simulated cropped image would be processed to have zero image intensities (i.e. black pixels/voxels, i.e. zero paddings) above or below the clipped anatomy coordinates, the ground-truth image will still contain, as metadata, the pixel-wise annotation for the entire liver, even though a portion of the liver is now simulated to be outside the FOV.

In the training phase for the machine learning model, the model could be presented with the cropped images as training input entries and the voxel-wise annotations for the full anatomies as ground truth entries, irrespective of the zero-padding in the cropped image regions.

The predicted probability masks obtained as the output from the model could be postprocessed for the estimation of the extent of the partially missing anatomy. This information could be used to recommend the optimized scan parameters (e.g. start and end positions of the scan range) to the user so that the partially missing anatomy is entirely covered in the second FOV in the second image data.

Fig. 9 illustrates the results of application of a machine learning algorithm which has been trained to perform the anatomy coverage check, in particular for the liver in this case, and to generate an estimate of an extension of the anatomy outside of the image boundary. Fig. 9(a) shows (dashed line) the Ground Truth annotation of the outer perimeter of a region containing the liver, where the region extends outside the boundary of the image. The actual image presented to the machine learning algorithm is the brighter region. Fig. 9(b) shows the output of the machine learning algorithm (solid line) when applied to this image, as compared with the Ground Truth (dashed line). The example illustrated is one test case with the liver truncated in the superior position. The network was trained using 2592 images. For purposes of this illustration, the coronal slice with the maximum number of voxels in ground truth is selected.

A similar algorithm could also be extended to 3D imaging, e.g. using the CT modality, including both high-dose and low-dose simulated 3D survey images.

Figs. 10-11 illustrate further results of application of a machine learning algorithm which has been trained to perform the anatomy coverage check, in this example for the target anatomy of the liver, where the training was specifically for CT imaging. The example of Fig. 10 utilized high dose CT images, and the example Fig. 11 utilized low dose CT images. The network was trained on approximately 850 high-dose and low-dose CT images individually. Fig. 10(a) and Fig. 11(a) each represent the Ground Truth (dashed line) annotation of the outer perimeter of the region containing the liver, where the region extends outside the boundary of the image. The actual image presented to the machine learning algorithm is the brighter region. Fig. 10(b) and Fig. 11(b) each show the output of the machine learning algorithm (solid line) when applied to this image, as compared with the Ground Truth (dashed line).

In the above outlined description, reference is made to use of a machine learning model comprising one or more machine learning algorithms.

A machine-learning algorithm is any self-training algorithm that processes input data in order to produce or predict output data. In the context of the above-described examples, the input data would comprise the first image data and the output data would comprise either: a binary classification as to whether a target anatomy is inside the FOV or not; or an estimate a spatial extension of the target anatomy outside of the boundary of the input image. If the first image data is 3D image data, the input to the machine learning model may be an extracted 2D slice from the 3D image data.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

In preferred examples, a deep-learning based artificial neural network may be used.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to the example cropped images of the target anatomy discussed above. The training output data entries correspond to the annotated segmented full versions of said cropped images.

Thus, in order to estimate the missing anatomical extent, a deep learning based image segmentation technique could be employed. The approach proposed herein differs from the known image segmentation methods since known image segmentation is usually restricted to segmenting anatomies in the given image FOV. In embodiments proposed herein, the segmentation of a partially missing anatomy is derived, where this can extend even beyond the image boundaries. This enables the estimated extent of the missing anatomy to be generated, and thus proposed adjustments to the imaging FOV derived.

The skilled person will know of numerous specific architectures suitable for the above-described purposes. By way of illustrative example, deep learning based image segmentation methods could be employed such as the U-Net architecture or Foveal-Net architecture. By way of further example, deep learning based object detection methods could be employed such as Mask R-CNN or RetinaNet. It would be possible to train such networks in an end-to-end fashion and could be integrated into the system with low computation resource required, and could even be run on a standard desktop CPU.

Details of the architecture of the U-Net segmentation network may be found for example in the following paper: O. Ronneberger, P. Fischer, and T. Brox, "U-Net: Convolutional Networks for Biomedical Image Segmentation," arXiv:1505.04597 [cs], May 2015.

Details of the architecture of the Foveal-Net segmentation network may be found for example in the following paper: Brosch, T. and Saalbach, A., "Foveal fully convolutional nets for multi-organ segmentation," in Medical Imaging 2018: Image Processing, Angelini, E. D. and Landman, B. A., eds., 10574, 198 - 206, International Society for Optics and Photonics, SPIE (2018).

Details of the architecture of the Mask R-CNN may be found in the following paper: Kaiming He, Georgia Gkioxari, Piotr Dollár, Ross Girshick, "Mask R-CNN", arXiv eprint 1703.06870, 2017.

Details of the architecture of the RetinaNet model may be found in the following paper: Tsung-Yi Lin and Priya Goyal and Ross Girshick and Kaiming He and Piotr Dollár, Focal Loss for Dense Object Detection, arXiv eprint 1708.02002, 2017.

By way of further illustration and explanation, one example of developing a machine learning model for use in accordance with one or more embodiments for segmentation of anatomies outside of the imaging FOV will now be outlined. This model makes use of an architecture known as F-Net. F-Net uses a multi-resolution approach while combining features of multiple scales, similar to the U-Net architecture mentioned above. However, while the U-Net model uses an encoder which comprises a consecutive series of filters, the F-Net model replaces the consecutive filters in the encoder by filters that operate on different image resolutions, thus resulting in a reduced number of neural network parameters. The segmentation of an image is achieved by consecutively segmenting non-overlapping 3D patches, which in turn are segmented by feeding in larger, overlapping patches at coarser scales in order to integrate contextual features. The features are extracted at each of a plurality of resolution levels using Case-based-reasoning (CBR) blocks consisting of a convolutional layer, batch normalization and rectified linear activation function. The feature maps of coarser resolutions are upsampled and integrated using CBR blocks, except at the finest level, where the convolutional layer is followed by a softmax layer to obtain the voxel-level class probabilities.

In the training phase, the network was presented with training data comprising a plurality of cropped images of a target anatomy as discussed above, and with the voxel-wise annotations providing the ground truth. Even though the low-level features such as intensities or texture carry the least relevant information for purposes of segmentation, it was investigated whether the high-level contextual features extracted at multiple resolution levels (as mentioned above) assist the network in learning how far the anatomy extends beyond the given FOV.

With such a network trained for a particular anatomy and a given test image, a probability map was obtained and processed to produce the binary segmentation mask using an optimal threshold calculated on the training dataset.

Finally, a bounding box tightly containing the binary segmentation mask was extracted for the evaluation and visualization purpose. The method was evaluated in terms of an extent-detection error which was computed as the distance between the most superior or inferior voxel in the GT and the network prediction, depending on the respective direction in which the anatomy was clipped.

Although in examples discussed above, the machine learning algorithm generates an output in the form of an estimated extension of an anatomy beyond the boundary of the image frame, additionally or alternatively, in further examples, the coverage check procedure may employ a machine learning algorithm adapted to provide a binary classification as to whether the target anatomy is fully covered by or contained within the first image data. As discussed above, a user interface may be controlled to provide a user output indicative of this determination. Various different options with regards to the interaction between the system and the user following a negative coverage check have already been discussed above.

Furthermore, although in examples discussed above, reference was made to a deep-learning based artificial neural network, other types of machine learning algorithm could be employed, such as for instance box-regression algorithms.

Furthermore, it is not essential to use a machine learning algorithm for the purpose of performing the coverage check. In other examples, model-based segmentation could be employed to achieve segmentation of the anatomy in the first image data. From the result of the segmentation it could be determined whether the whole of an anatomy of interest is covered in the first FOV. The skilled person in this field will know of numerous model-based segmentation approaches which employ classical algorithmic approaches such as shape-based object detection. Another possible approach to performing the coverage check is to perform mapping of the first image data to a probabilistic anatomic atlas. One example of this approach is described for instance in the following paper: Astrid Franz, et al "Annotation-free probabilistic atlas learning for robust anatomy detection in CT images", Proc. SPIE 9413, Medical Imaging 2015: Image processing, 941338 (20 March 2015).

The anatomical image analysis and the coverage check could be performed as separate steps or could both be performed by a single algorithm or model. For example a machine learning model could be trained to receive the first image data as an input and to generate the coverage check result as the output, wherein the anatomical image analysis (e.g. segmentation) is inherently performed as part of the analysis achieved by the model. In other examples, it is possible to use a first algorithm or model which performs segmentation, the output of which is an anatomical segmentation of the first image data, and a second algorithm or model, the output of which is a result of the coverage check, where this could be a binary classification (covered or not covered), or could be an indication of an extension of the anatomy beyond the first FOV in one or more dimensions.

It is also possible to train a single multi-task network with two outputs: one output being a segmentation which gives an indication for instance of an extent of the anatomy extending beyond the FOV and the other output being a binary classification indicative of whether the anatomy is covered or not covered.

Additionally or alternatively to the various features outlined above, in accordance with one or more embodiments, the method may further comprise applying the coverage check operation to the acquired second image data. A user interface may be controlled to display a result of this further coverage check. This could be applied for example to only a subset of the second image data, e.g. just one or more image slices from the second image data. This check could be performed as a precaution, so that in case some part of the target anatomy has still been missed in one or more of the image frames (e.g. because the patient shifted position during the scan), acquisition can be repeated before the subject leaves, saving time in rescheduling a further imaging scan.

For example, in the context of CT imaging, in a typical imaging workflow, each 2D slice is displayed on the user interface display screen as a preview image after the acquisition of a diagnostic CT image (i.e. the second image data). The coverage check operation could thus be applied to these 2D preview images for investigating whether the target anatomy to be imaged is included in the FOV. In case of any images delivering a negative coverage check result, an alert may be generated using the user interface, and optionally along with a proposed adjustment to the scan protocol, e.g. start and end position of the scan range, in order to ensure the target anatomy is included in the FOV. A new diagnostic image could then be acquired before releasing the patient, thus avoiding patient recall.

A further aspect of the invention provides a computer program product comprising code means configured, when executed by a processor, the processor being operatively coupled to a medical imaging apparatus, to cause the processor to perform a method in accordance with any method outlined in this disclosure.

Embodiments of the invention described above employ a processing arrangement. The processing arrangement may in general comprise a single processor or a plurality of processors. It may be located in a single containing device, structure or unit, or it may be distributed between a plurality of different devices, structures or units. Reference therefore to the processing arrangement being adapted or configured to perform a particular step or task may correspond to that step or task being performed by any one or more of a plurality of processing components, either alone or in combination. The skilled person will understand how such a distributed processing arrangement can be implemented. The processing arrangement includes a communication module or input/output for receiving data and outputting data to further components.

The one or more processors of the processing arrangement can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer implemented method comprising:
receiving first image data (72, 74) of a patient anatomy from a medical imaging apparatus (10), wherein the first image data (72, 74) is 2D or 3D image data, and wherein the first image data (72, 74) is data acquired in accordance with a first scan protocol comprising a first set of scan parameters for the medical imaging apparatus (10), wherein the first scan protocol is for acquisition of image data which covers a first FOV;
applying anatomical image analysis to the first image data (72, 74) to detect at least a portion (80) of a defined target anatomy in the image data;
performing a coverage check comprising determining from the image analysis whether the defined target anatomy is fully contained within the first image data (72, 74);
generating a data representation of the result of the coverage check; and **characterized in that** the method further comprises:
determining a second scan protocol defining a second set of scan parameters for the imaging apparatus (10), and wherein the second scan protocol is for acquiring image data which covers a second FOV, wherein the second FOV is the same as or different to the first FOV;
controlling a user interface (30, 32) to display an indication of the result of the coverage check;
receiving a user input from the user interface (30, 32) following display of the coverage check, and wherein the second FOV of the second scan protocol is determined in dependence upon the user input; and
acquiring second image data of the patient anatomy in accordance with the second scan protocol, the second image data covering the second FOV, wherein the second image data is 2D or 3D image data.

2. The method of claim 1, wherein the data representation of the result of the coverage check is communicated to a datastore for storage of the result of the coverage check; and wherein the second image data is stored in the same or a different datastore, associated with the result of the coverage check.

3. The method of claim 1, wherein the method further comprises:
responsive to a negative result of the coverage check, determining a proposed adjustment to the first scan protocol so as to acquire an extended FOV, wherein the proposed adjustment is based on the anatomical image analysis; and
either
setting the second scan protocol in accordance with the proposed adjusted first scan protocol, such that the second FOV is set as the extended FOV; or
communicating the proposed adjustment to the first scan protocol and/or the proposed extended FOV to the user interface (30, 32).

4. The method of any of claims 1-3, further comprising applying the anatomical image analysis to estimate a spatial extension (84) of the target anatomy beyond at least one boundary of the FOV, and optionally further comprising controlling a user interface (30, 32) to display a visual representation of said spatial extension (84).

5. The method of claim 4, wherein the coverage check comprises determining, based on the spatial extension (84) of the target anatomy beyond the FOV, a proposed adjustment to the first scan protocol for acquiring an extended FOV which fully covers the target anatomy, and
optionally wherein the second scan protocol is determined such that the second FOV is set as the extended FOV.

6. The method of claim 5, further comprising:
controlling a user interface (30, 32) to display a representation of the proposed adjustment to the first scan protocol and/or proposed extended FOV;
generating a prompt on the user interface (30, 32) requesting user approval;
receiving a user input from the user interface (30, 32) indicative of approval or non-approval, and
acquiring second image data in accordance with the proposed adjusted scan protocol, spanning the extended FOV, only responsive to receipt of a user input indicative of approval.

7. The method of claim 5, further comprising:
controlling a user interface (30, 32) to display a visual representation of the proposed extended FOV relative to a rendered view of the first image data (72, 74);
controlling the user interface (30, 32) to generate a prompt for a user input indicative of approval of the proposed extended FOV, or indicative of an amendment to the proposed extended FOV, via operation of a user control; and
determining the second scan protocol such that the second FOV is set as the extended FOV, responsive to receipt of user approval from the user-interface (30, 32); OR
determining the second scan protocol such that the second FOV is set as a user-amended FOV, wherein the user-amended FOV is defined based on a received user input indicative of an amendment to the extended FOV.

8. The method of any of claims 5-7, wherein estimating the spatial extension (84) of the target anatomy outside of the FOV comprises estimating an outline of a boundary of at least the portion of the target anatomy which lies outside of the FOV, and wherein the method further comprises generating a visual depiction of said outline relative to the first image data (72, 74) on a display of a user interface (30, 32).

9. The method of any of claims 1-8, wherein the scan parameters of the first and second scan protocols include boundaries of a scan range along at least one scan axis of the medical imaging apparatus (10), and wherein the first FOV and second FOV are each defined at least in part by the boundaries of said scan range.

10. The method of any of claims 1-9, wherein
the data representation is communicated to a datastore for storage of the result of the coverage check;
and wherein the method further comprises performing a quality assessment comprising deriving a quality indicator for the acquired second image data based on the result of the coverage check associated with the image data.

11. The method of any of claims 1-10, wherein performing the coverage check comprises application of a machine learning model, and wherein the machine learning model is configured to
receive, as an input, image data representing only a portion of an anatomical object, and
generate, as an output, an estimate of at least a dimensional extent of a remainder of the anatomical object outside a field of the image data; and/or
generate, as an output, an estimation of a boundary (88) of the remainder of the anatomical object outside a field of the image data.

12. The method of claim 11, wherein the machine learning model is trained using training data entries which each comprise
a first data input for the model comprising a cropped image of the anatomy in which a section of the image has been removed so that the image depicts only a portion of the anatomical object, and
a ground truth for the model comprising at least voxel-wise anatomical labelling for voxels of a second, non-cropped version of the image, which depicts the whole of the anatomical object.

13. A computer program product comprising code means configured, when executed by a processor (24), the processor (24) being operatively coupled to a medical imaging apparatus (10), to cause the processor (24) to perform a method in accordance with any of claims 1-12.

14. A processing arrangement (20) comprising:
an input/output (22) for connecting in use to a medical imaging apparatus (10); and
one or more processors (24) adapted to:
receive, at the input/output (22), first image data (72, 74) of a patient anatomy from a medical imaging apparatus (10), wherein the first image data (72, 74) is 2D or 3D image data, and wherein the first image data (72, 74) is data acquired in accordance with a first scan protocol comprising a first set of scan parameters for the imaging apparatus (10), wherein the first scan protocol is for acquisition of image data which covers a first FOV;
apply anatomical image analysis to the first image data (72, 74) to detect at least a portion (80) of a defined target anatomy in the image data;
perform a coverage check comprising determining from the image analysis whether the defined target anatomy is fully contained within the first image data (72, 74);
generate a data representation of the result of the coverage check;
**characterized in that** the one or more processors (24) are further adapted to:
determine a second scan protocol defining a second set of scan parameters for the imaging apparatus (10), and wherein the second scan protocol is for acquiring image data which covers a second FOV, wherein the second FOV is the same as or different to the first FOV;
control a user interface (30, 32) to display an indication of the result of the coverage check;
receive a user input from the user interface (30, 32) following display of the coverage check, and wherein the second FOV of the second scan protocol is determined in dependence upon the user input; and
output at the input/output (22) a control signal for controlling the imaging apparatus (10) to acquire second image data of the patient anatomy in accordance with the second scan protocol, the second image data covering the second FOV, wherein the second image data is 2D or 3D image data.

15. A system, comprising:
a medical imaging apparatus (10); and
a processing arrangement (20) as claimed in claim 14.

## Patentansprüche

1. Computerimplementiertes Verfahren, umfassend:
Empfangen erster Bilddaten (72, 74) einer Patientenanatomie von einer medizinischen Bildgebungseinrichtung (10), wobei die ersten Bilddaten (72, 74) 2D- oder 3D-Bilddaten sind, und wobei die ersten Bilddaten (72, 74) Daten sind, die in Übereinstimmung mit einem ersten Scanprotokoll erfasst werden, das einen ersten Satz von Scanparametern für die medizinische Bildgebungseinrichtung (10) umfasst, wobei das erste Scanprotokoll zum Erfassen von Bilddaten dient, die ein erstes Sichtfeld, FOV, abdecken;
Anwenden einer anatomischen Bildanalyse auf die ersten Bilddaten (72, 74), um mindestens einen Abschnitt (80) einer definierten Zielanatomie in den Bilddaten zu erkennen;
Durchführen einer Abdeckungsprüfung, die das Bestimmen aus der Bildanalyse umfasst, ob die definierte Zielanatomie vollständig in den ersten Bilddaten (72, 74) enthalten ist;
Erzeugen einer Datendarstellung des Ergebnisses der Abdeckungsprüfung; und
**dadurch gekennzeichnet, dass** das Verfahren weiter umfasst:
Bestimmen eines zweiten Scanprotokolls, das einen zweiten Satz von Scanparametern für die Bildgebungseinrichtung (10) definiert, und wobei das zweite Scanprotokoll zum Erfassen von Bilddaten dient, die ein zweites FOV abdecken, wobei das zweite FOV gleich dem ersten FOV ist oder sich davon unterscheidet;
Steuern einer Benutzeroberfläche (30, 32), um eine Angabe des Ergebnisses der Abdeckungsprüfung anzuzeigen;
Empfangen einer Benutzereingabe von der Benutzeroberfläche (30, 32) nach dem Anzeigen der Abdeckungsprüfung, und wobei das zweite FOV des zweiten Scanprotokolls in Abhängigkeit von der Benutzereingabe bestimmt wird; und
Erfassen zweiter Bilddaten der Patientenanatomie in Übereinstimmung mit dem zweiten Scanprotokoll, wobei die zweiten Bilddaten das zweite FOV abdecken, wobei die zweiten Bilddaten 2D- oder 3D-Bilddaten sind.

2. Verfahren nach Anspruch 1, wobei die Datendarstellung des Ergebnisses der Abdeckungsprüfung zum Speichern des Ergebnisses der Abdeckungsprüfung an einen Datenspeicher kommuniziert wird; und wobei die zweiten Bilddaten im selben oder einem unterschiedlichen Datenspeicher, der dem Ergebnis der Abdeckungsprüfung zugeordnet ist, gespeichert werden.

3. Verfahren nach Anspruch 1, wobei das Verfahren weiter umfasst:
als Reaktion auf ein negatives Ergebnis der Abdeckungsprüfung, Bestimmen einer vorgeschlagenen Anpassung an das erste Scanprotokoll, um ein erweitertes FOV zu erfassen, wobei die vorgeschlagene Anpassung auf der anatomischen Bildanalyse basiert; und
entweder
Festlegen des zweiten Scanprotokolls in Übereinstimmung mit dem vorgeschlagenen angepassten ersten Scanprotokoll derart, dass das zweite FOV als das erweiterte FOV festgelegt wird; oder
Kommunizieren der vorgeschlagenen Anpassung an das erste Scanprotokoll und/oder des vorgeschlagenen erweiterten FOVs an die Benutzeroberfläche (30, 32).

4. Verfahren nach einem der Ansprüche 1-3, das weiter das Anwenden der anatomischen Bildanalyse umfasst, um eine räumliche Erstreckung (84) der Zielanatomie über mindestens eine Grenze des FOVs hinaus zu schätzen, und das optional weiter das Steuern einer Benutzeroberfläche (30, 32) umfasst, um eine visuelle Darstellung der räumlichen Erstreckung (84) anzuzeigen.

5. Verfahren nach Anspruch 4, wobei die Abdeckungsprüfung das Bestimmen basierend auf der räumlichen Erstreckung (84) der Zielanatomie über das FOV hinaus, einer vorgeschlagenen Anpassung an das erste Scanprotokoll zum Erfassen eines erweiterten FOV umfasst, das die Zielanatomie vollständig abdeckt, und
wobei das zweite Scanprotokoll optional bestimmt wird, sodass das zweite FOV als das erweiterte FOV festgelegt wird.

6. Verfahren nach Anspruch 5, weiter umfassend:
Steuern einer Benutzeroberfläche (30, 32), um eine Darstellung der vorgeschlagenen Anpassung an das erste Scanprotokoll und/oder des vorgeschlagenen erweiterten FOV anzuzeigen;
Erzeugen einer Aufforderung auf der Benutzeroberfläche (30, 32), die eine Benutzergenehmigung erfordert;
Empfangen einer Benutzereingabe von der Benutzeroberfläche (30, 32), die eine Genehmigung oder Nichtgenehmigung angibt, und
Erfassen zweiter Bilddaten in Übereinstimmung mit dem vorgeschlagenen angepassten Scanprotokoll, welches das erweiterte FOV überspannt, nur als Reaktion auf das Empfangen einer Benutzereingabe, die eine Genehmigung angibt.

7. Verfahren nach Anspruch 5, weiter umfassend:
Steuern einer Benutzeroberfläche (30, 32), um eine visuelle Darstellung des vorgeschlagenen erweiterten FOV in Bezug zu einer gerenderten Ansicht der ersten Bilddaten (72, 74) anzuzeigen;
Steuern der Benutzeroberfläche (30, 32), um über die Bedienung einer Benutzerssteuerung eine Aufforderung für eine Benutzereingabe zu erzeugen, welche die Genehmigung des vorgeschlagenen erweiterten FOV angibt oder eine Änderung des vorgeschlagenen erweiterten FOV angibt; und
Bestimmen des zweiten Scanprotokolls, sodass das zweite FOV als das erweiterte FOV festgelegt wird, als Reaktion auf den Empfang einer Benutzergenehmigung von der Benutzeroberfläche (30, 32); ODER
Bestimmen des zweiten Scanprotokolls, sodass das zweite FOV als ein von dem Benutzer geändertes FOV festgelegt wird, wobei das von dem Benutzer geänderte FOV basierend auf einer empfangenen Benutzereingabe definiert wird, die eine Änderung des erweiterten FOV angibt.

8. Verfahren nach einem der Ansprüche 5-7, wobei Schätzen der räumlichen Erstreckung (84) der Zielanatomie außerhalb des FOVs Schätzen eines Umrisses einer Grenze mindestens des Abschnitts der Zielanatomie umfasst, der außerhalb des FOVs liegt, und wobei das Verfahren weiter Erzeugen einer visuellen Abbildung des Umrisses in Bezug zu den ersten Bilddaten (72, 74) auf einer Anzeige einer Benutzeroberfläche (30, 32) umfasst.

9. Verfahren nach einem der Ansprüche 1-8, wobei die Scanparameter des ersten und des zweiten Scanprotokolls Grenzen eines Scanbereichs entlang mindestens einer Scanachse der medizinischen Bildgebungseinrichtung (10) beinhalten, und wobei das erste FOV und das zweite FOV jeweils mindestens zum Teil durch die Grenzen des Scanbereichs definiert sind.

10. Verfahren nach einem der Ansprüche 1-9, wobei
die Datendarstellung an einen Datenspeicher zum Speichern des Ergebnisses der Abdeckungsprüfung kommuniziert wird;
und wobei das Verfahren weiter Durchführen einer Qualitätsbeurteilung umfasst, die Ableiten eines Qualitätsindikators für die erfassten zweiten Bilddaten basierend auf dem Ergebnis der den Bilddaten zugeordneten Abdeckungsprüfung umfasst.

11. Verfahren nach einem der Ansprüche 1-10, wobei Durchführen der Abdeckungsprüfung umfasst
Anwendung eines maschinellen Lernmodells, und wobei das maschinelle Lernmodell konfiguriert ist, um als eine Eingabe Bilddaten zu empfangen, die nur einen Abschnitt eines anatomischen Objekts darstellen, und
als eine Ausgabe eine Schätzung von mindestens einer dimensionalen Erstreckung eines Rests des anatomischen Objekts außerhalb eines Feldes der Bilddaten zu erzeugen; und/oder
als eine Ausgabe eine Schätzung einer Grenze (88) des Rests des anatomischen Objekts außerhalb eines Feldes der Bilddaten zu erzeugen.

12. Verfahren nach Anspruch 11, wobei das maschinelle Lernmodell unter Verwendung von Trainingsdateneinträgen trainiert wird, die jeweils umfassen
eine erste Dateneingabe für das Modell, die ein zugeschnittenes Bild der Anatomie umfasst, in dem eine Sektion des Bildes entfernt worden ist, sodass das Bild nur einen Abschnitt des anatomischen Objekts abbildet, und
eine Grundwahrheit für das Modell, die mindestens eine voxelweise anatomische Beschriftung für Voxel einer zweiten, nicht zugeschnittenen Version des Bildes umfasst, welche die Gesamtheit des anatomischen Objekts abbildet.

13. Computerprogrammprodukt, das Codemittel umfasst, die so konfiguriert sind, dass sie, wenn sie durch einen Prozessor (24) ausgeführt werden, wobei der Prozessor (24) mit einer medizinischen Bildgebungseinrichtung (10) operativ gekoppelt ist, den Prozessor (24) veranlassen, ein Verfahren nach einem der Ansprüche 1-12 durchzuführen.

14. Verarbeitungsanordnung (20), umfassend:
einen Eingang/Ausgang (22) zum Verbinden bei Verwendung mit einer medizinischen Bildgebungseinrichtung (10); und
einen oder mehrere Prozessoren (24), die angepasst sind, um:
am Eingang/Ausgang (22) erste Bilddaten (72, 74) einer Patientenanatomie von einer medizinischen Bildgebungseinrichtung (10) zu empfangen, wobei die ersten Bilddaten (72, 74) 2D- oder 3D-Bilddaten sind, und wobei die ersten Bilddaten (72, 74) Daten sind, die in Übereinstimmung mit einem ersten Scanprotokoll erfasst werden, das einen ersten Satz von Scanparametern für die Bildgebungseinrichtung (10) umfasst, wobei das erste Scanprotokoll zum Erfassen von Bilddaten dient, die ein erstes FOV abdecken;
anatomische Bildanalyse auf die ersten Bilddaten (72, 74) anzuwenden, um mindestens einen Abschnitt (80) einer definierten Zielanatomie in den Bilddaten zu erkennen;
eine Abdeckungsprüfung durchzuführen, die das Bestimmen aus der Bildanalyse umfasst, ob die definierte Zielanatomie vollständig in den ersten Bilddaten (72, 74) enthalten ist;
eine Datendarstellung des Ergebnisses der Abdeckungsprüfung zu erzeugen;
**dadurch gekennzeichnet, dass** der eine oder mehrere Prozessoren (24) weiter angepasst sind, um:
ein zweites Scanprotokoll, das einen zweiten Satz von Scanparametern für die Bildgebungseinrichtung (10) definiert, zu bestimmen, und wobei das zweite Scanprotokoll zum Erfassen von Bilddaten dient, die ein zweites FOV abdecken, wobei das zweite FOV gleich dem ersten FOV ist oder sich davon unterscheidet;
eine Benutzeroberfläche (30, 32) zu steuern, um eine Angabe des Ergebnisses der Abdeckungsprüfung anzuzeigen;
eine Benutzereingabe von der Benutzeroberfläche (30, 32) nach Anzeigen der Abdeckungsprüfung zu empfangen, und wobei das zweite FOV des zweiten Scanprotokolls in Abhängigkeit von der Benutzereingabe bestimmt wird; und
am Eingang/Ausgang (22) ein Steuersignal zum Steuern der Bildgebungseinrichtung (10) auszugeben, um zweite Bilddaten der Patientenanatomie in Übereinstimmung mit dem zweiten Scanprotokoll zu erfassen, wobei die zweiten Bilddaten das zweite FOV abdecken, wobei die zweiten Bilddaten 2D- oder 3D-Bilddaten sind.

15. System, umfassend:
eine medizinische Bildgebungseinrichtung (10); und
eine Verarbeitungsanordnung (20) nach Anspruch 14.

## Revendications

1. Procédé mis en œuvre par ordinateur, comprenant :
la réception de premières données d'image (72, 74) d'une anatomie de patient en provenance d'un appareil d'imagerie médicale (10), dans lequel les premières données d'image (72, 74) sont des données d'image 2D ou 3D et dans lequel les premières données d'image (72, 74) sont des données acquises selon un premier protocole de balayage comprenant un premier ensemble de paramètres de balayage pour l'appareil d'imagerie médicale (10), dans lequel le premier protocole de balayage est destiné à une acquisition de données d'image qui couvrent un premier champ de vision, FOV ;
l'application d'une analyse d'image anatomique aux premières données d'image (72, 74) pour détecter au moins une partie (80) d'une anatomie cible définie dans les données d'image ;
la réalisation d'une vérification de couverture comprenant la détermination à partir de l'analyse d'image que l'anatomie cible définie est entièrement contenue dans les premières données d'image (72, 74) ;
la génération d'une représentation de données du résultat de la vérification de couverture ; et
**caractérisé en ce que** le procédé comprend en outre :
la détermination d'un second protocole de balayage définissant un second ensemble de paramètres de balayage pour l'appareil d'imagerie (10) et dans lequel le second protocole de balayage est destiné à acquérir des données d'image qui couvrent un second FOV, dans lequel le second FOV est identique au FOV ou différent de celui-ci ;
la commande d'une interface utilisateur (30, 32) pour afficher une indication du résultat de la vérification de couverture ;
la réception d'une entrée d'utilisateur en provenance de l'interface utilisateur (30, 32) après l'affichage de la vérification de couverture et dans lequel le second FOV du second protocole de balayage est déterminé en fonction de l'entrée d'utilisateur ; et
l'acquisition de secondes données d'image de l'anatomie de patient selon le second protocole de balayage, les secondes données d'image couvrant le second FOV, dans lequel les secondes données d'image sont des données d'image 2D ou 3D.

2. Procédé selon la revendication 1, dans lequel la représentation de données du résultat de la vérification de couverture est communiquée à un magasin de données pour un stockage du résultat de vérification de couverture ; et dans lequel les secondes données d'image sont stockées dans le même magasin de données ou dans un magasin de données différent, associé au résultat de la vérification de couverture.

3. Procédé selon la revendication 1, dans lequel le procédé comprend en outre :
à la suite d'un résultat négatif de la vérification de couverture, la détermination d'un ajustement proposé au premier protocole de balayage de sorte à acquérir un FOV étendu, dans lequel l'ajustement proposé est basé sur l'analyse de l'image anatomique ; et
soit
la définition du second protocole de balayage selon le premier protocole de balayage ajusté proposé de telle sorte que le second FOV soit défini comme FOV étendu ; soit
la communication de l'ajustement proposé au premier protocole de balayage et/ou du FOV étendu proposé à l'interface utilisateur (30, 32).

4. Procédé selon l'une quelconque des revendications 1-3, comprenant en outre l'application de l'analyse d'image anatomique pour estimer une extension spatiale (84) de l'anatomie cible au-delà d'au moins une limite du FOV et, facultativement, comprenant en outre la commande d'une interface utilisateur (30, 32) pour afficher une représentation visuelle de ladite extension spatiale (84).

5. Procédé selon la revendication 4, dans lequel la vérification de couverture comprend la détermination, sur la base de l'extension spatiale (84) de l'anatomie cible au-delà du FOV, d'un ajustement proposé au premier protocole de balayage pour acquérir un FOV étendu qui couvre entièrement l'anatomie cible, et
facultativement dans lequel le second protocole de balayage est déterminé de telle sorte que le second FOV soit défini comme le FOV étendu.

6. Procédé selon la revendication 5, comprenant en outre :
la commande d'une interface utilisateur (30, 32) pour afficher une représentation de l'ajustement proposé au premier protocole de balayage et/ou au FOV étendu proposé ;
la génération d'une invite sur l'interface utilisateur (30, 32) demandant une approbation d'utilisateur ;
la réception d'une entrée d'utilisateur en provenance de l'interface utilisateur (30, 32) indiquant une approbation ou une non-approbation, et
l'acquisition de secondes données d'image selon le protocole de balayage ajusté proposé, couvrant le FOV étendu, uniquement en réponse à la réception d'une entrée d'utilisateur indiquant une approbation.

7. Procédé selon la revendication 5, comprenant en outre :
la commande d'une interface utilisateur (30, 32) pour afficher une représentation visuelle du FOV étendu proposé par rapport à une vue restituée des premières données d'image (72, 74) ;
la commande de l'interface utilisateur (30, 32) pour générer une invite pour une entrée d'utilisateur indiquant l'approbation du FOV étendu proposé, ou indiquant une modification du FOV étendu proposé, via l'opération d'une commande d'utilisateur ; et
la détermination du second protocole de balayage de telle sorte que le second FOV soit défini comme le FOV étendu, à la suite de la réception d'une approbation d'utilisateur en provenance de l'interface utilisateur (30, 32) ; OU
la détermination du second protocole de balayage de telle sorte que le second FOV soit défini comme un FOV modifié par un utilisateur, dans lequel le FOV modifié par un utilisateur est défini sur la base d'une entrée d'utilisateur reçue indiquant une modification du FOV étendu.

8. Procédé selon l'une quelconque des revendications 5-7, dans lequel l'estimation de l'extension spatiale (84) de l'anatomie cible à l'extérieur du FOV comprend l'estimation d'un contour d'une limite d'au moins la partie de l'anatomie cible qui se trouve à l'extérieur du FOV, et dans lequel le procédé comprend en outre la génération d'une représentation visuelle dudit contour par rapport aux premières données d'image (72, 74) sur un affichage d'une interface utilisateur (30, 32).

9. Procédé selon l'une quelconque des revendications 1-8, dans lequel les paramètres de balayage des premier et second protocoles de balayage incluent des limites d'une plage de balayage le long d'au moins un axe de balayage de l'appareil d'imagerie médicale (10) et dans lequel le premier FOV et le second FOV sont chacun définis au moins en partie par les limites de ladite plage de balayage.

10. Procédé selon l'une quelconque des revendications 1-9, dans lequel
la représentation de données est communiquée à un magasin de données pour un stockage du résultat de la vérification de couverture ;
et dans lequel le procédé comprend en outre la réalisation d'une évaluation de qualité comprenant la déduction d'un indicateur de qualité pour les secondes données d'image acquises sur la base du résultat de la vérification de couverture associé aux données d'image.

11. Procédé selon l'une quelconque des revendications 1-10, dans lequel la réalisation de la vérification de couverture comprend
l'application d'un modèle d'apprentissage automatique et dans lequel le modèle d'apprentissage automatique est configuré pour recevoir, comme entrée, des données d'image représentant uniquement une partie d'un objet anatomique, et
la génération, comme sortie, d'une estimation d'au moins une étendue dimensionnelle d'un reste de l'objet anatomique en dehors d'un champ des données d'image ; et/ou
la génération, comme sortie, d'une estimation d'une limite (88) du reste de l'objet anatomique en dehors d'un champ des données d'image.

12. Procédé selon la revendication 11, dans lequel le modèle d'apprentissage automatique est formé à l'aide d'entrées de données d'entraînement qui comprennent mutuellement
une première entrée de données pour le modèle comprenant une image recadrée de l'anatomie dans laquelle une section de l'image a été supprimée de telle sorte que l'image ne représente qu'une partie de l'objet anatomique, et
une réalité de terrain pour le modèle comprenant au moins un étiquetage anatomique par voxel pour des voxels d'une seconde version non recadrée de l'image, qui représente l'ensemble de l'objet anatomique.

13. Produit de programme informatique comprenant des moyens de code configurés, lorsqu'ils sont exécutés par un processeur (24), le processeur (24) étant couplé de manière fonctionnelle à un appareil d'imagerie médicale (10), pour amener le processeur (24) à réaliser un procédé selon l'une quelconque des revendications 1-12.

14. Agencement de traitement (20), comprenant :
une entrée/sortie (22) pour être raccordée lors de l'utilisation à un appareil d'imagerie médicale (10) ; et
un ou plusieurs processeurs (24) adaptés pour :
recevoir, au niveau de l'entrée/sortie (22), des premières données d'image (72, 74) d'une anatomie de patient en provenance d'un appareil d'imagerie médicale (10), dans lequel les premières données d'image (72, 74) sont des données d'image 2D ou 3D et dans lequel les premières données d'image (72, 74) sont des données acquises selon un premier protocole de balayage comprenant un premier ensemble de paramètres de balayage pour l'appareil d'imagerie (10), dans lequel le premier protocole de balayage est destiné à une acquisition de données d'image qui couvrent un premier FOV ;
appliquer une analyse d'image anatomique aux premières données d'image (72, 74) pour détecter au moins une partie (80) d'une anatomie cible définie dans les données d'image ;
réaliser une vérification de couverture comprenant la détermination à partir de l'analyse d'image que l'anatomie cible définie est entièrement contenue dans les premières données d'image (72, 74) ;
générer une représentation de données du résultat de la vérification de couverture ;
**caractérisé en ce que** les un ou plusieurs processeurs (24) sont en outre adaptés pour :
déterminer un second protocole de balayage définissant un second ensemble de paramètres de balayage pour l'appareil d'imagerie (10) et dans lequel le second protocole de balayage est destiné à acquérir des données d'image qui couvrent un second FOV, dans lequel le second FOV est identique au premier FOV ou différent de celui-ci ;
commander une interface utilisateur (30, 32) pour afficher une indication du résultat de la vérification de couverture ;
recevoir une entrée d'utilisateur en provenance de l'interface utilisateur (30, 32) après l'affichage du contrôle de couverture et dans lequel le second FOV du second protocole de balayage est déterminé en fonction de l'entrée d'utilisateur ; et
émettre, au niveau de l'entrée/sortie (22), un signal de commande pour commander l'appareil d'imagerie (10) pour acquérir des secondes données d'image de l'anatomie du patient selon le second protocole de balayage, les secondes données d'image couvrant le second FOV, dans lequel les secondes données d'image sont des données d'image 2D ou 3D.

15. Système, comprenant :
un appareil d'imagerie médicale (10) ; et
un agencement de traitement (20) selon la revendication 14.
